# EUROPEAN PATENT APPLICATION

(11) **EP 4 016 341 A1**
(43) Date of publication of application: **22.06.2022**
(21) Application number: 20853186.3
(22) Date of filing: 12.08.2020
(51) Int. Cl.: G06F 21/32, B60K 28/06, A61B 5/1171

(54) **RECOGNITION SYSTEM, RECOGNITION METHOD, RECOGNITION PROGRAM, AND COMPUTER-READABLE NON-TRANSITORY STORAGE MEDIUM**

(30) Priority: 13.08.2019 JP 2019148513
(71) Applicant: Tanita Corporation, Tokyo 174-8630 (JP)
(72) Inventor: MOCHIZUKI Kei, Tokyo 174-8630 (JP); SAGAWA Kiyoshi, Tokyo 174-8630 (JP); KASAHARA Yasuhiro, Tokyo 174-8630 (JP); IKEDA Satoshi, Tokyo 174-8630 (JP); SASAKI Shinji, Tokyo 174-8630 (JP)
(74) Representative: Eisenführ Speiser
(86) International application number: PCT/JP2020/030648
(87) International publication number: WO 2021/029418

(57) **Abstract**

In an alcohol detection system, face authentication is performed on a person to be measured before an alcohol detection device starts measurement, and the success of the face authentication performed on the person to be measured triggers the acquisition of camera image information that are images of the face of the person to be measured continuously shot by a camera. In other words, when the face authentication performed on the person to be measured succeeds, the person to be measured starts alcohol detection and images of the face of the person to be measured are continuously shot during the measurement.

## Description

### CROSS-REFERENCE TO RELATED APPLICATION

This application claims the benefit of Japanese Patent Application No. 2019-148513 filed on August 13, 2019 in Japan, the contents of which are incorporated herein by reference.

### TECHNICAL FIELD

The present invention relates to an authentication system, an authentication method, an authentication program, and a computer-readable non-transitory storage medium.

### BACKGROUND ART

Alcohol testing using alcohol detectors is recently widely performed on drivers and crew members of business vehicles, aircrafts, ships, railroad trains, or the like at roll call or the like. When an alcohol test is performed, frauds are prevented generally by an on-site checker (an observer) checking a driver or a crew member, who is a person to be measured, against a photograph of the face of the person to be measured during alcohol measurement done by the person to be measured. Roll call, however, is sometimes performed remotely in such a way that a driver or a crew member is in a remote location and a checker does the check via a monitor or the like. In such a case, a tablet terminal or another terminal device is used to send a photograph of the face of a person to be measured themself, and therefore frauds may be committed that include measurement performed by another person impersonating the person to be measured and measurement with a different device (a dummy device).

In this regard, Patent document 1 (Japanese Patent Laid-Open Application No. 2013-192859) includes a biological information measuring system comprising: a biological information measuring device provided with a light-emitting unit; a first terminal device provided with a camera; and a second terminal device for face recognition processing to authenticate a person or the like, for the purpose of effectively preventing fraudulent measurement using a dummy device and another person than a person to be measured.

In this biological information measuring system, a measurer operates an input unit of the second terminal device to cause a light emission instruction signal indicating a light emission pattern, that the second terminal device has been caused to determine, to be sent from the second terminal device to the biological information measuring device before measurement with the biological information measuring device is started. The camera then keeps on shooting the light-emitting unit emitting light according to the light emission pattern and the face of a person to be measured, and stores the shot data in a storage of the first terminal device. The second terminal device determines whether the light emitted from the light-emitting unit visible in the shot data shot by the camera is in accordance with the light emission pattern or not, and determines whether face data generated from the shot data and a registered face image represent the face of the same person or not.

### SUMMARY OF THE INVENTION

### Problems to be solved by the invention

Patent document 1 discloses that, in the biological information measuring system, the light-emission result distinction process and the face recognition process are performed before the person to be measured blows the breath into the biological information measuring device and are then also performed during the blowing.

In the biological information measuring system of Patent document 1, however, the camera performs the retake when the person to be measured is detected blowing the breath into a mouthpiece, and therefore a period during which the camera does not shoot occurs between the shooting with the camera for the first face recognition and the shooting with the camera during the blowing. That is, if swapping of the person to be measured or other fraudulent acts are committed during this period during which the camera does not shoot, those acts cannot be recognized.

Shooting the face of the person to be measured with the camera at inappropriate times in this manner may cause not being able to prevent the person to be measured from committing fraudulent acts.

A purpose of the invention made in view of the above is to provide an authentication system, an authentication method, an authentication program, and a computer-readable non-transitory storage medium that can prevent a person to be measured who measures biological information from committing fraudulent acts.

### Means for solving the problems

An authentication system of an aspect of the disclosure comprises: a biological information measuring device for measuring biological information of a person to be measured; an image-shooting device; and an information-processing device, where the information-processing device is provided with face authentication means for performing face authentication on the person to be measured based on image information that indicates a face of the person to be measured an image of which has been shot by the image-shooting device, and where the image-shooting device acquires image information that are continuously shot images of the face of the person to be measured on a condition that the face authentication succeeds before the biological information measuring device starts the measurement.

In this configuration, face authentication is performed on the person to be measured before the biological information measuring device starts the measurement, and image information that are continuously shot images of the face of the person to be measured are acquired on a condition that the face authentication performed on the person to be measured succeeds. In other words, when the face authentication performed on the person to be measured succeeds, the person to be measured starts to measure biological information and images of the face of the person to be measured are continuously shot during the measurement. Consequently, this configuration allows preventing the person to be measured who measures biological information from committing fraudulent acts.

In the above-described authentication system, the face authentication means performs the face authentication before the biological information measuring device is powered on, and the biological information measuring device being powered on after the success of the face authentication triggers the image-shooting device to acquire image information that are continuously shot images of the face of the person to be measured.

This configuration allows the acquisition of image information, that are continuously shot images of the face of the person to be measured, at an appropriate time when the measurement of biological information is started.

In the above-described authentication system, the image-shooting device finishes the continuous image-shooting after the biological information measuring device finishes the measurement, when acquisition of an object to be measured, which is a subject of the measurement, is started, or when the acquisition of the object to be measured is finished.

This configuration allows preventing the person to be measured who measures biological information from committing fraudulent acts during a period before the biological information measuring device finishes the measurement.

In the above-described authentication system, the image-shooting device acquires image information that is a shot image of a measurement value of the biological information displayed on the biological information measuring device.

This configuration allows a measurement value of the biological information to be stored as image information.

In the above-described authentication system, the biological information measuring device is provided with display means for displaying identification information, the image-shooting device shoots an image of the identification information displayed on the display means along with the face of the person to be measured, and the information-processing device uses identification information authentication means to perform authentication based on the identification information an image of which has been shot by the image-shooting device, and uses the face authentication means to perform authentication based on the face of the person to be measured an image of which has been shot by the image-shooting device.

This configuration allows two types of authentication to be performed based on the identification information and the face of the person to be measured, and therefore allows preventing the person to be measured who measures biological information from committing fraudulent acts. The identification information is displayed on the display means as, for example, a two-dimensional code such as a QR code (registered trademark).

In the above-described authentication system, the image-shooting device acquires image information that are continuously shot images of the face of the person to be measured if the authentication based on the identification information and the authentication based on the face of the person to be measured succeed.

This configuration allows two types of authentication to be performed based on the identification information and the face of the person to be measured, and therefore allows preventing the person to be measured who measures biological information from committing fraudulent acts.

In the above-described authentication system, the information-processing device generates the identification information each time the biological information measuring device performs the measurement, and the biological information measuring device causes the display means to display the identification information based on the identification information generated by the information-processing device.

This configuration prevents the person to be measured from getting to know the identification information before the measurement, and therefore allows preventing frauds on the identification information.

In the above-described authentication system, the authentication based on the identification information is performed at least one of: before the biological information measuring device starts the measurement; while the biological information measuring device is performing the measurement; and after the biological information measuring device finishes the measurement.

This configuration can more reliably prevent the person to be measured who measures biological information from committing fraudulent acts.

In the above-described authentication system, the biological information measuring device displays the identification information including information on a measurement result on the display means after finishing the measurement, and the image-shooting device acquires image information that is a shot image of the identification information displayed on the display means.

This configuration allows a result of measuring biological information with the biological information measuring device to be confirmed also by image information acquired by the image-shooting device.

In the above-described authentication system, the biological information of the person to be measured is breath alcohol concentration of the person to be measured.

This configuration allows determination of whether the measurement is performed by the person to be measured themself who performs alcohol detection or not with simpler components.

In the above-described authentication system, the face authentication means performs the face authentication based on a plurality of image information.

This configuration allows the face authentication to be performed by using a plurality of image information, and therefore can more reliably prevent the person to be measured who measures biological information from committing fraudulent acts.

In the above-described authentication system, the face authentication means performs face authentication on the person to be measured with the image information and registered image information that is registered in advance and indicates the face of the person to be measured.

This configuration allows the face authentication to be performed by using registered image information that is registered in advance, and therefore can more reliably prevent the person to be measured who measures biological information from committing fraudulent acts.

In the above-described authentication system, the biological information of the person to be measured is measured during a predetermined time period.

This configuration allows the biological information of the person to be measured to be measured around an intended time period.

In the above-described authentication system, the person to be measured is a crew member of an aircraft, and the predetermined time period is at least one of specified time periods before and after an operation time period of the aircraft that the crew member boards for service.

This configuration allows the biological information of the crew member of the aircraft to be measured around the operation time of the aircraft.

An authentication system of an aspect of the disclosure comprises: a device to be used that requires authentication for use; an image-shooting device; and an information-processing device, where the device to be used is provided with display means for displaying identification information, where the image-shooting device shoots an image of the identification information displayed on the display means along with a face of a user of the device to be used, and where the information-processing device performs authentication based on the identification information and the face of the user an image of which has been shot by the image-shooting device.

This configuration allows two types of authentication to be performed based on the identification information and the face of the user, and therefore allows preventing the user who uses the device to be used from committing fraudulent acts.

An authentication method of an aspect of the disclosure is of a system comprising: a biological information measuring device for measuring biological information of a person to be measured; an image-shooting device; and an information-processing device, and the authentication method has: a first step of the information-processing device performing face authentication on the person to be measured based on image information that indicates a face of the person to be measured an image of which has been shot by the image-shooting device; and a second step of the image-shooting device acquiring image information that are continuously shot images of the face of the person to be measured on a condition that the face authentication succeeds before the biological information measuring device starts the measurement.

An authentication method of an aspect of the disclosure is of a system comprising: a biological information measuring device for measuring biological information of a person to be measured; an image-shooting device; and an information-processing device, and the authentication method has: a first step of the biological information measuring device displaying identification information on display means; a second step of the image-shooting device shooting an image of the identification information displayed on the display means along with a face of the person to be measured, and a third step of the information-processing device using identification information authentication means to perform authentication based on the identification information an image of which has been shot by the image-shooting device, and using face authentication means to perform authentication based on the face of the person to be measured an image of which has been shot by the image-shooting device.

An authentication program of an aspect of the disclosure is of a system comprising: a biological information measuring device for measuring biological information of a person to be measured; an image-shooting device; and an information-processing device, and the authentication program causes a computer to execute: a first step of the information-processing device performing face authentication on the person to be measured based on image information that indicates a face of the person to be measured an image of which has been shot by the image-shooting device; and a second step of the image-shooting device acquiring image information that are continuously shot images of the face of the person to be measured on a condition that the face authentication succeeds before the biological information measuring device starts the measurement.

An authentication program of an aspect of the disclosure is of a system comprising: a biological information measuring device for measuring biological information of a person to be measured; an image-shooting device; and an information-processing device, and the authentication program causes a computer to execute: a first step of the biological information measuring device displaying identification information on display means; a second step of the image-shooting device shooting an image of the identification information displayed on the display means along with a face of the person to be measured, and a third step of the information-processing device using identification information authentication means to perform authentication based on the identification information an image of which has been shot by the image-shooting device, and using face authentication means to perform authentication based on the face of the person to be measured an image of which has been shot by the image-shooting device.

### ADVANTAGE OF THE INVENTION

The disclosure allows preventing the person to be measured who measures biological information from committing fraudulent acts.

### BRIEF DESCRIPTION OF THE DRAWINGS

Figure 1 is a schematic configuration view of an alcohol detection system of a first embodiment;
Figure 2 is a function block diagram of the alcohol detection system of the first embodiment;
Figure 3 is a schematic diagram showing alcohol detection time periods of the first embodiment;
Figure 4 is a flowchart of an alcohol detection process of the first embodiment;
Figure 5 is a schematic configuration view of an alcohol detection system of the first embodiment;
Figure 6 is a function block diagram of an alcohol detection system of a second embodiment;
Figure 7 is a flowchart of an alcohol detection process of the second embodiment;
Figure 8 is a flowchart of an alcohol detection process of a third embodiment;
Figure 9 is a flowchart of an alcohol detection process of a variation; and
Figure 10 shows a schematic configuration of an alcohol detection system of a variation.

### MODES OF EMBODYING THE INVENTION

Embodiments will now be described with reference to the drawings. The embodiments described below are merely illustrative of ways to implement the disclosure, and do not limit the disclosure to the specific configurations described below. When the disclosure is to be implemented, any specific configuration may be appropriately adopted according to the embodiment.

### (First embodiment)

An alcohol detection system is taken as an example of the authentication system in this embodiment. That is to say, the biological information is the breath alcohol concentration of a person to be measured and the biological information measuring device is an alcohol detection device in the embodiment. A pilot or a flight attendant, who is a crew member of an aircraft, is taken as an example of a person to be measured in the embodiment. Examples of the aircraft include, but are not limited to, a passenger plane and a cargo plane, and it may be a business jet or another aircraft.

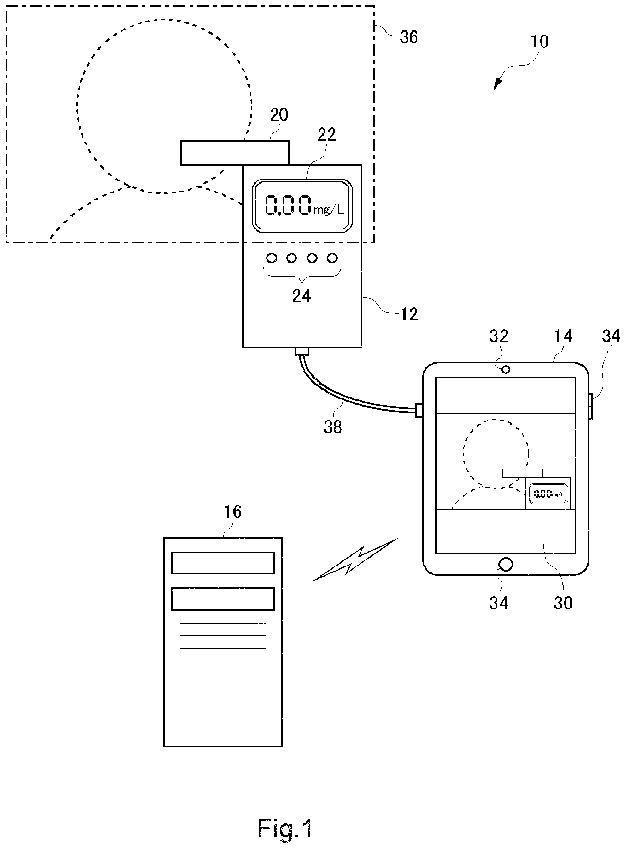

Figure 1 is a schematic configuration view of an alcohol detection system 10 of the embodiment. The alcohol detection system 10 comprises an alcohol detection device 12, a tablet terminal 14, and an authentication server 16. Note that each person to be measured has their own alcohol detection device 12 and tablet terminal 14 in the embodiment for example, but the embodiment is not limited to this, and a plurality of persons to be measured may share one alcohol detection device 12 with one another or a plurality of persons to be measured may share one tablet terminal 14 with one another.

The alcohol detection device 12 performs measurement of the alcohol concentration in the breath of a person to be measured (hereinafter also referred to as "alcohol detection"), and comprises a mouthpiece 20, a display 22, and an operation unit 24. The mouthpiece 20 is formed with an air inlet on one end and an air outlet on the other end. When a person to be measured holds the air inlet in the person's mouth and exhales into it, the alcohol concentration in the exhalation blown into the mouthpiece 20 is measured, and the blown breath is released from the air outlet.

The display 22 displays a result of the measurement of the alcohol concentration in the breath blown by a person to be measured (hereinafter referred to as an "alcohol measurement value") and the like. The operation unit 24 is operated by a person to be measured, and comprises a power button for powering on and off the alcohol detection device 12, a measurement start button for starting alcohol detection, a setup button for changing various settings and the like, and the like.

The tablet terminal 14 is a portable information-processing device, and comprises a computer such as a CPU (Central Processing Unit) capable of executing a program, a storage device such as an SSD (Solid State Drive), a communication device for connecting to the Internet or an intranet, various types of connectors, a touch-panel display 30 for displaying an image and accepting an operation for the tablet terminal 14, a camera 32 for shooting an image of a subject, and an operation unit 34 for accepting an operation for the tablet terminal 14.

The tablet terminal 14 of the embodiment is held by a person to be measured who is a crew member of an aircraft. The tablet terminal 14, for example, holds a flight schedule of an aircraft that the crew member boards for service and is also used to manage the crew member's work. As described in detail later, a person to be measured performs alcohol detection based on a flight schedule.

The tablet terminal 14 receives an alcohol measurement value and the like sent from the alcohol detection device 12. When a person to be measured performs alcohol detection using the alcohol detection device 12, the camera 32 shoots an image so that the face of the person to be measured and the display 22 are included in the angle of view 36, and displays the shot image on the touch-panel display 30.

The tablet terminal 14 then sends the alcohol measurement value, and image information acquired by the camera 32 shooting the image (hereinafter referred to as "camera image information") to the authentication server 16. The tablet terminal 14 also receives a result of authentication of the person to be measured based on the camera image information, and displays them on the touch-panel display 30.

Note that the tablet terminal 14 is installed with an application software for performing an alcohol detection process using the alcohol detection device 12 (hereinafter referred to as the "alcohol detection app"). When performing alcohol detection, a person to be measured starts the alcohol detection app on the tablet terminal 14, performs alcohol detection according to a procedure instructed by the alcohol detection app, and determines whether the person to be measured is allowed to board for service or not based on the result of the alcohol detection. In addition to the alcohol detection procedure, the alcohol detection app also causes an authentication result received from the authentication server 16 and the like to be displayed on the touch-panel display 30.

The authentication server 16 is an information-processing device comprising a computer such as a CPU capable of executing a program, a storage device such as an HDD (Hard Disk Drive), a communication device for connecting to the Internet or an intranet, various types of connectors, and the like. The authentication server 16 of the embodiment performs identification of a person to be measured and other various types of information processing. It holds information required for identification beforehand, and sequentially holds a measurement result (an alcohol measurement value) obtained by the alcohol detection device 12 and the like in association with a person to be measured as described in detail later.

The authentication server 16 is connected to, for example, an information-processing device of a higher level for managing a crew member, a flight schedule, or the like (hereinafter referred to as a "management server"), an information-processing device used by a manager of a crew member (hereinafter referred to as a "management terminal"), or the like. This allows the authentication server 16 to send information based on alcohol detection to a management server or send to a predetermined mail address a result of an alcohol detection process described in detail later, or more specifically, failure of authentication or detection of a fraud. Upon viewing such a mail on a management terminal, a manager deals with the relevant crew member.

In the alcohol detection system 10 of the embodiment, the alcohol detection device 12 and the tablet terminal 14 send and receive information between each other via, for example, wire communication using a USB (Universal Serial Bus) cable 38. The communication method, however, is not limited to this, and information may be sent and received via, for example, Bluetooth (registered trademark) or other short-range wireless communication. The tablet terminal 14 and the authentication server 16 send and receive information between each other via, for example, the Internet or an intranet using Wi-Fi or other wireless communication.

Now, the alcohol detection system 10 of the embodiment makes identification for determining whether a person to be measured themself is performing alcohol detection using the alcohol detection device 12 or not. In other words, the alcohol detection system 10 of the embodiment prevents a person other than a person to be measured themself from pretending to be the person to be measured to fraudulently perform alcohol detection.

For this purpose, in the alcohol detection system 10 of the embodiment, the authentication server 16 performs face authentication on a person to be measured based on camera image information that indicates the face of the person to be measured an image of which has been shot by the camera 32 before alcohol detection is started. The camera 32 then acquires camera image information that are continuously shot images of the face of the person to be measured on a condition that the face authentication succeeds before alcohol detection is started.

As described above, in the alcohol detection system 10 of the embodiment, a person to be measured starts alcohol detection and the camera 32 continuously shoots images of the face of the person to be measured during the measurement when face authentication performed on the person to be measured succeeds. This allows the camera 32 to continue the image-shooting (the acquisition of camera image information) without interruption after the first face authentication succeeds, and therefore it is difficult to commit swapping of the person to be measured or other fraudulent acts after the success of the face authentication. Consequently, the alcohol detection system 10 of the embodiment can prevent a person to be measured who performs alcohol detection from committing fraudulent acts. In the embodiment, the success of face authentication triggers the camera 32 to start to continuously shoot images of the face of a person to be measured.

Note that the term "trigger" means to start continuous image-shooting with the camera 32 (hereinafter referred to as "continuous shooting") at the starting point when face authentication succeeds. The alcohol detection system 10 may start continuous shooting not only almost immediately after the success of face authentication, but also, for example, after a specified time has passed since the success of face authentication, as long as the condition that is the success of face authentication is satisfied. The alcohol detection system 10 may determine whether another predetermined condition is satisfied before the face authentication or not and the success of face authentication after the condition is satisfied may trigger continuous shooting.

A system in which, for example, face authentication and alcohol detection are performed at the same time is not time-effective and wastes the time and effort of a person to be measured, since face authentication and alcohol detection have to be performed at the same time again if face authentication fails. Alcohol detection being performed after the success of face authentication as in the alcohol detection system 10 of the embodiment, on the other hand, can reduce time inefficiency and the time and effort of a person to be measured due to a failure of face authentication, since only face authentication has to be performed again if face authentication fails.

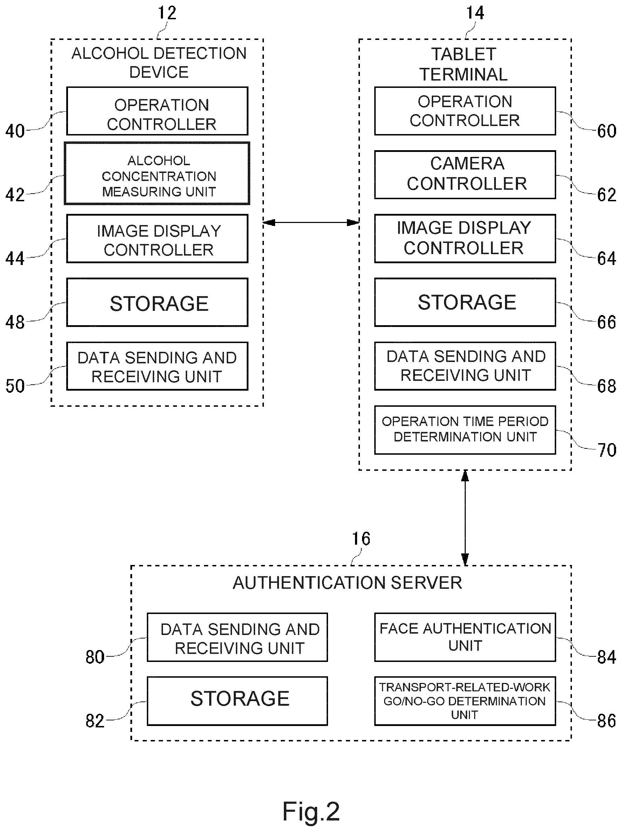

Figure 2 is a function block diagram for an alcohol detection process performed in the alcohol detection system 10 of the embodiment. Functions shown in Figure 2 in the embodiment are executed by, for example, computers each comprised in the alcohol detection device 12, the tablet terminal 14, and the authentication server 16. The configuration is not limited to this, and the functions may be implemented by ASICs (Application Specific Integrated Circuits) or other specific hardware each comprised in the alcohol detection device 12, the tablet terminal 14, and the authentication server 16.

The alcohol detection device 12 comprises an operation controller 40, an alcohol concentration measuring unit 42, an image display controller 44, a storage 48, and a data sending and receiving unit 50.

The operation controller 40 accepts an operation for the operation unit 24 done by a person to be measured, and provides an instruction for control according to the operation, such as on/off or the like of the alcohol detection device 12 or the start of alcohol detection.

The alcohol concentration measuring unit 42 measures the alcohol concentration in a breath blown through the mouthpiece 20 after the measurement start button included in the operation unit 24 is operated, and outputs the result as an alcohol measurement value.

The image display controller 44 controls the display state of the display 22 in such a way as to cause the display 22 to display an alcohol measurement value.

The storage 48 holds the manufacturing ID of the alcohol detection device 12, the setting status of the alcohol detection device 12, and the like. The manufacturing ID is an ID that is set at the time of manufacture of the alcohol detection device 12.

The data sending and receiving unit 50 sends and receives information to and from the tablet terminal 14 and another information-processing device through wire or wireless communication. The data sending and receiving unit 50 of the embodiment sends an alcohol measurement value, the manufacturing ID, and the like to the tablet terminal 14.

The tablet terminal 14 comprises an operation controller 60, a camera controller 62, an image display controller 64, a storage 66, a data sending and receiving unit 68, and an operation time period determination unit 70.

The operation controller 60 accepts an operation for the touch-panel display 30 and the operation unit 34 done by a person to be measured, and performs control according to the operation, such as execution of the alcohol detection app.

The camera controller 62 controls the camera 32 according to an operation done by a person to be measured.

The image display controller 64 controls the image display on the touch-panel display 30.

The storage 66 holds the alcohol detection app, camera image information, a flight schedule of a person to be measured who owns the tablet terminal 14, and the like. The flight schedule is sent from another information-processing device, and is automatically updated as required.

The data sending and receiving unit 68 sends and receives information to and from the alcohol detection device 12, the authentication server 16, and another information-processing device through wire or wireless communication. The data sending and receiving unit 68 of the embodiment receives an alcohol measurement value from the alcohol detection device 12, and sends these information to the authentication server 16 along with camera image information. The data sending and receiving unit 68 also receives an authentication result and the like from the authentication server 16.

The operation time period determination unit 70 determines a time period for alcohol detection based on a flight schedule stored in the storage 66. That is, the alcohol detection system 10 of the embodiment measures the alcohol concentration in the breath of a person to be measured in a predetermined time period. This allows the alcohol detection system 10 to measure the alcohol concentration of a person to be measured in an intended time period, that is, around the time when the person to be measured boards an aircraft for service in the embodiment.

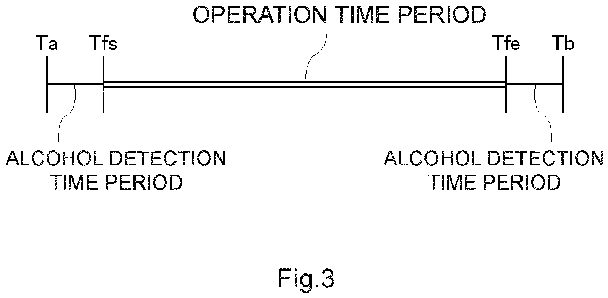

Figure 3 is a schematic diagram showing alcohol detection time periods of the embodiment. The operation time period shown in Figure 3 is an operation time of an aircraft that a person to be measured boards for service. In alcohol detection of the embodiment, a person to be measured performs alcohol detection within specified time periods before and after this operation time period.

In the example in Figure 3, time from a time Ta to an operation start time Tfs and time from an operation end time Tfe to a time Tb are set to be alcohol detection time periods. An interval between the operation start time Tfs and the time Ta and an interval between the operation end time Tfe and the time Tb are arbitrarily determined and are, for example, an hour. There may be a rule stating that a person to be measured should perform alcohol detection in at least one of time periods before and after an operation time period of an aircraft that the person to be measured boards for service. For example, the rule may state that a flight attendant should perform alcohol detection before and after an operation time period and a pilot, on the other hand, should perform alcohol detection only before an operation time period. If operating hours are to be long as in the case with an international flight or the like, the rule may state that alcohol detection should be performed within a specified time period during the operation time period.

The authentication server 16 comprises a data sending and receiving unit 80, a storage 82, a face authentication unit 84, and a transport-related-work go/no-go determination unit 86.

The data sending and receiving unit 80 receives an alcohol measurement value, camera image information, and the like sent from the tablet terminal 14, and sends an authentication result and the like to the tablet terminal 14.

The storage 82 holds a person-to-be-measured list and a device list. On the person-to-be-measured list, various pieces of registration information including the name, affiliation, and the like of a person to be measured are registered along with image information indicating the face of the person to be measured (hereinafter referred to as "registered face image information") for each registered person. On the device list, the manufacturing IDs of the alcohol detection devices 12 used in the alcohol detection system 10 are registered. Which alcohol detection device 12 a person to be measured is to use may be determined in advance, and in that case the manufacturing ID of the alcohol detection device 12 to be used by the person to be measured is also registered on the person-to-be-measured list.

The storage 82 also holds for each person to be measured an alcohol measurement value, camera image information, and the like sent from the tablet terminal 14.

The face authentication unit 84 of the embodiment performs face authentication on a person to be measured based on face image information that is indicated by camera image information and indicates the face of the person to be measured. The face authentication unit 84 of the embodiment performs the face authentication on a person to be measured with face image information that is indicated by camera image information and indicates the face of the person to be measured and registered face image information that is registered in advance on the person-to-be-measured list. The face authentication unit 84 also has a function to extract the face of a person to be measured from camera image information to acquire face image information.

The transport-related-work go/no-go determination unit 86 determines the state of alcohol ingestion of a person to be measured based on an alcohol measurement value and a predetermined reference value and, based on the determination, determines whether the person to be measured can be allowed to board for service or not. That is, if the alcohol measurement value exceeds the reference value, the relevant person to be measured is prohibited from boarding for service. Incidentally, the transport-related-work go/no-go determination unit 86 may be comprised in the tablet terminal 14.

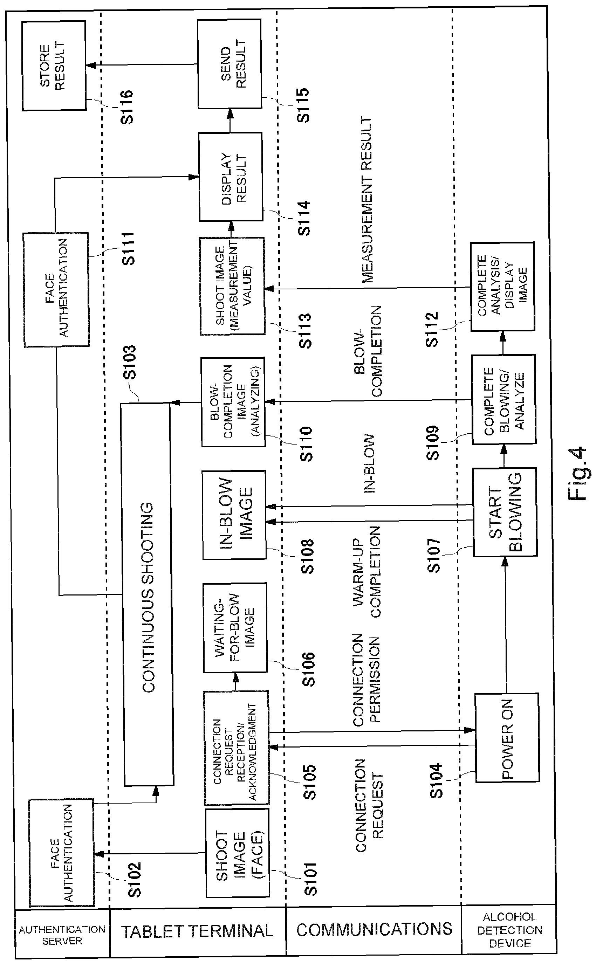

Figure 4 is a flowchart of an alcohol detection process (an alcohol detection program) of the embodiment. The alcohol detection process shown in Figure 4 is started by starting the alcohol detection app installed in the tablet terminal 14. The alcohol detection program may be provided to the alcohol detection system 10 by the alcohol detection system 10 (the alcohol detection device 12, the tablet terminal 14, and the authentication server 16) downloading it from a communication network, or may be provided to the alcohol detection system 10 via a non-transitory recording medium.

First, the camera controller 62 of the tablet terminal 14 controls the camera 32 so as to shoot an image of the face of a person to be measured, and acquires camera image information (a step S101). The image shot by the camera 32 is displayed on the touch-panel display 30 of the tablet terminal 14 during the shooting, and the person to be measured adjusts the position of the person themself so that the face of the person themself is included in the angle of view 36 of the camera 32. The acquisition of camera image information just mentioned means storing the image shot by the camera 32 in a non-volatile storage device (e.g., the storage 66) comprised in the tablet terminal 14. The acquired camera image information is sent to the authentication server 16.

The face authentication unit 84 of the authentication server 16 then performs face authentication based on the camera image information sent to the authentication server 16 (a step S102). The face authentication of the embodiment is performed on the person to be measured, for example, with face image information that indicates the face of the person to be measured and registered face image information that is registered on the person-to-be-measured list. The alcohol detection process determines that a login is performed and proceeds to a step S103 if the face authentication succeeds, but causes the touch-panel display 30 of the tablet terminal 14 to display an image that indicates a failure of authentication if the face authentication fails.

Note that the success of face authentication in the step S102 allows the alcohol detection app (the tablet terminal 14) to recognize that the person to be measured starts alcohol detection.

Then, on the condition that the face authentication succeeds, the camera controller 62 of the tablet terminal 14 controls the camera 32 so as to acquire camera image information that are continuously shot images (images obtained by continuous shooting) of the face of the person to be measured (the step S103). The images shot by the camera 32 are also displayed on the touch-panel display 30 of the tablet terminal 14 during the shooting, and the person to be measured adjusts the position of the person themself so that the face of the person themself is included in the angle of view 36 of the camera 32.

Continuous shooting in the embodiment is performed in such a way that the camera 32 acquires a still image every 500 msec, and all the acquired still images are stored in a non-volatile storage device (the storage 66).

If the face authentication succeeds in the step S102, an image urging to powering on the alcohol detection device 12 is displayed on the touch-panel display 30 of the tablet terminal 14. Upon viewing this image, the person to be measured presses the power button of the alcohol detection device 12 to power on the alcohol detection device 12 (a step S104).

When powered on, the alcohol detection device 12 sends a connection request to the tablet terminal 14. Upon receiving the connection request, the tablet terminal 14 sends a connection permission to the alcohol detection device 12 (a step S105) as an acknowledgment to it. A connection between the alcohol detection device 12 and the tablet terminal 14 is established by the alcohol detection device 12 receiving the connection permission.

The tablet terminal 14 then causes the touch-panel display 30 to display a waiting-for-blow image (a step S106). The waiting-for-blow image is an image that urges the person to be measured to blow the breath into the alcohol detection device 12, and is displayed, for example, along with a countdown of a specified time (e.g., five seconds) that is the same as the warm-up time of the alcohol detection device 12.

When the countdown is complete, a warm-up completion signal indicating the completion of the warm-up is sent from the alcohol detection device 12 to the tablet terminal 14, and when the person to be measured starts blowing the breath, a blow-start signal indicating that the blowing is started is sent to the tablet terminal 14 (a step S107).

Upon receiving the blow-start signal, the tablet terminal 14 causes the touch-panel display 30 to display a blow-in-progress image (a step S108). The blow-in-progress image is an image that allows the person to be measured to recognize that alcohol detection is being performed.

When the person to be measured finishes blowing the breath for a specified time, a solenoid valve of the alcohol detection device 12 is activated to cause a pump to draw a specified amount of breath into the device, and an alcohol measurement value is calculated by the alcohol concentration measuring unit 42 analyzing the breath (a step S109). The alcohol detection device 12 sends a blow-completion signal indicating the completion of the blowing of the breath to the tablet terminal 14.

Upon receiving the blow-completion signal, the tablet terminal 14 causes the touch-panel display 30 to display a blow-completion image (a step S110). The blow-completion image is an image that allows the person to be measured to recognize that the alcohol concentration is being analyzed.

When the tablet terminal 14 receives the blow-completion signal, the camera controller 62 controls the camera 32 so as to finish the continuous shooting. A plurality of camera image information acquired by the continuous shooting are sent to the authentication server 16. When the authentication server 16 receives the camera image information acquired by the continuous shooting, the face authentication unit 84 performs face authentication based on the camera image information acquired by the continuous shooting (a step S111). The determination result of the face authentication is sent to the tablet terminal 14. Note that all the camera image information acquired by the continuous shooting do not have to be used for the face authentication, and at least one of the camera image information may be chosen for the face authentication.

As seen above, the alcohol detection process of the embodiment allows face authentication to be performed by using a plurality of camera image information, and therefore can more reliably prevent a person to be measured who performs alcohol detection from committing fraudulent acts. In addition, since the camera 32 finishes continuous shooting after the alcohol detection is finished, the alcohol detection process of the embodiment can prevent the person to be measured from committing fraudulent acts during a period before the alcohol detection is finished. Note that the expression "after the alcohol detection is finished" can be restated as "after the analysis of alcohol concentration is finished."

The camera 32 may finish continuous shooting not only after the alcohol detection is finished, but also when the acquisition of an object to be measured which is a subject of the measurement is started or when the acquisition of the object to be measured is finished. The object to be measured in the embodiment is the breath of a person to be measured. That is, the camera 32 may finish continuous shooting when the person to be measured starts blowing the breath into the alcohol detection device 12 or when the person to be measured finishes blowing the breath. Note that when the blowing of the breath is started is, for example, when a pressure sensor of the alcohol detection device 12 detects pressure fluctuations, and when the blowing of the breath is finished is, for example, when the solenoid valve of the alcohol detection device 12 is activated.

Camera image information may be sent sequentially to the authentication server 16 during continuous shooting, and the face authentication in the step S111 may be performed not after but before the continuous shooting is finished. Moreover, the face authentication may be performed not by comparing face image information with registered face image information, but by comparing a plurality of camera image information acquired by the continuous shooting with one another and determining whether face image information included in the camera image information belong to an identical person or not.

In an example of the alcohol detection process of the embodiment, the face authentication determining whether face image information belong to an identical person or not is not performed by comparing camera image information with one another that are acquired during the display of the blow-in-progress image displayed in the step S108 and the blow-completion image displayed in the step S110, that is, from when the person to be measured blows the breath into the alcohol detection device 12 until when the blowing is finished. This is because it is unlikely that the person to be measured changes during the blowing of the breath. Another reason for the face authentication not to be performed by comparing camera image information with one another is that an error will result if the blowing becomes undetectable after once the blowing from the person to be measured is detected (the pressure sensor detects pressure fluctuations) and before the blowing is finished. That is, an attempt of the person to be measured to change during the blowing of the breath causes the blowing to be undetectable, resulting in an error, which also allows a fraud to be detected. The embodiment is not limited to this, and the face authentication may be performed by comparing camera image information with one another that are acquired from when the person to be measured blows the breath into the alcohol detection device 12 until when the blowing is finished.

When the analysis of the breath alcohol concentration is completed, then the alcohol detection device 12 displays an alcohol measurement value, which is the analysis result, on the display 22, and sends the alcohol measurement value (the measurement result) to the tablet terminal 14 (a step S112). When the tablet terminal 14 receives the alcohol measurement value, the camera 32 acquires camera image information that is a shot image of the alcohol measurement value displayed on the display 22 of the alcohol detection device 12 (a step S113).

Since the camera 32 of the embodiment acquires camera image information that is a shot image of an alcohol measurement value displayed on the alcohol detection device 12 as described above, the alcohol measurement value can be stored as image information.

Incidentally, for example, the alcohol detection process of the embodiment does not involve face authentication based on camera image information acquired by the image-shooting in the step S113 but makes the authentication server 16 store the camera image information as proof of an alcohol detection result. The embodiment, however, is not limited to this, and face authentication may be performed based on the camera image information acquired in the step S113.

The tablet terminal 14 also causes the touch-panel display 30 to display the face authentication result and the alcohol measurement value, causing the person to be measured to recognize them as the result of the alcohol detection (a step S114). In so doing, the tablet terminal 14 also displays determination results made by the operation time period determination unit 70 and the transport-related-work go/no-go determination unit 86 of the authentication server 16 on the touch-panel display 30. The tablet terminal 14 then sends the alcohol measurement value sent from the alcohol detection device 12, the camera image information including the alcohol measurement value acquired in the step S113, the camera image information acquired by the continuous shooting in the step S103, other determination results, and the like to the authentication server 16 (a step S115).

Upon receiving the various pieces of information sent by the tablet terminal 14 in the step S115, the authentication server 16 stores (saves) them in association with identification information that indicates the person to be measured (a step S116). This is the end of the alcohol detection process of the embodiment.

While in the alcohol detection process of the embodiment the camera 32 starts continuous shooting on the condition that the face authentication succeeds, the embodiment is not limited to this, and there may be another condition for starting the continuous shooting as well as the condition that face authentication succeeds. That is, the continuous shooting with the camera 32 has only to be started after face authentication succeeds and before the person to be measured starts alcohol measurement using the alcohol detection device 12.

For example, in the alcohol detection process, the face authentication unit 84 may perform face authentication (the step S102) before the alcohol detection device 12 is powered on, and the alcohol detection device 12 being powered on (the step S104) after the success of the face authentication may trigger the camera 32 to perform continuous shooting. That is, in this embodiment, the condition for starting the continuous shooting is the success of the face authentication and the alcohol detection device 12 being powered on, and therefore the continuous shooting in the step S103 is preformed after the alcohol detection device 12 issues a connection request to the tablet terminal 14 (the step S105).

This mode allows the acquisition of camera image information, that are continuously shot images of the face of the person to be measured, at an appropriate time when alcohol measurement is started. That is, the person to be measured is not yet ready for alcohol detection before the alcohol detection device 12 is powered on. For this reason, the use of camera image information acquired by continuous shooting in such a state for face authentication would be inappropriate for identification of the person to be measured, and therefore the continuous shooting may be performed after the alcohol detection device 12 is powered on.

In an alcohol detection process of another embodiment, the camera 32 may start the continuous shooting when, after the success of the face authentication in the step S102, the alcohol detection device 12 is powered on and a countdown of a specified time (e.g., five seconds) is complete. That is, the condition for starting the continuous shooting in this embodiment is the success of the face authentication, the alcohol detection device 12 being powered on, and the completion of the countdown. Note that the countdown is for a time required for the person to be measured to prepare to start the blowing into the alcohol detection device 12, and is set in advance.

In an alcohol detection process of another embodiment, the alcohol detection device 12 seen from a plurality of angles may be captured by the continuous shooting. For example, the person to be measured may be guided by means of a sound or an image after the start of the continuous shooting and before the blowing of the breath so as to make a plurality of sides of the alcohol detection device 12 face toward the camera 32. This can prevent a fraud committed by performing alcohol detection with the alcohol detection device 12 that is fraudulently altered. The time when the alcohol detection device 12 seen from a plurality of angles is captured by the continuous shooting is not limited to this, and the capture may be performed during or after the blowing of the breath.

### (Second embodiment)

A second embodiment is described below.

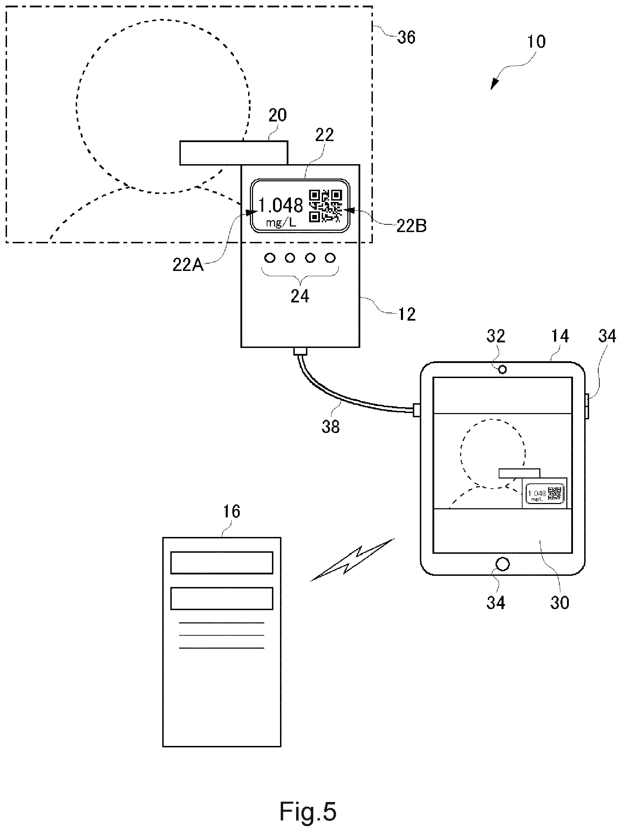

Figure 5 is a schematic configuration view of the alcohol detection system 10 of the embodiment. The same components in Figure 5 as in Figure 1 are designated by the same symbols, and their descriptions are omitted.

The alcohol detection device 12 of the embodiment displays an identification information (hereinafter referred to as an "indication ID") on the display 22. The alcohol detection device 12 of the embodiment has a measurement value display area 22A and an ID display area 22B in the display 22, and displays an alcohol measurement value in the measurement value display area 22A and an indication ID in the ID display area 22B.

As described in detail later, an indication ID of the embodiment is sent from the tablet terminal 14 and is displayed, for example, as a QR code or another two-dimensional code in the ID display area 22B.

As seen above, the alcohol detection device 12 of the embodiment displays an indication ID on the display 22. The camera 32 then shoots an image of the indication ID displayed on the display 22 along with the face of a person to be measured, and the authentication server 16 performs authentication based on the indication ID an image of which has been shot by the camera 32, and performs authentication based on the face of the person to be measured. The alcohol detection system 10 of the embodiment thus allows two types of authentication to be performed based on the indication ID and the face of the person to be measured, and therefore allows more reliably preventing the person to be measured who performs alcohol detection from committing fraudulent acts.

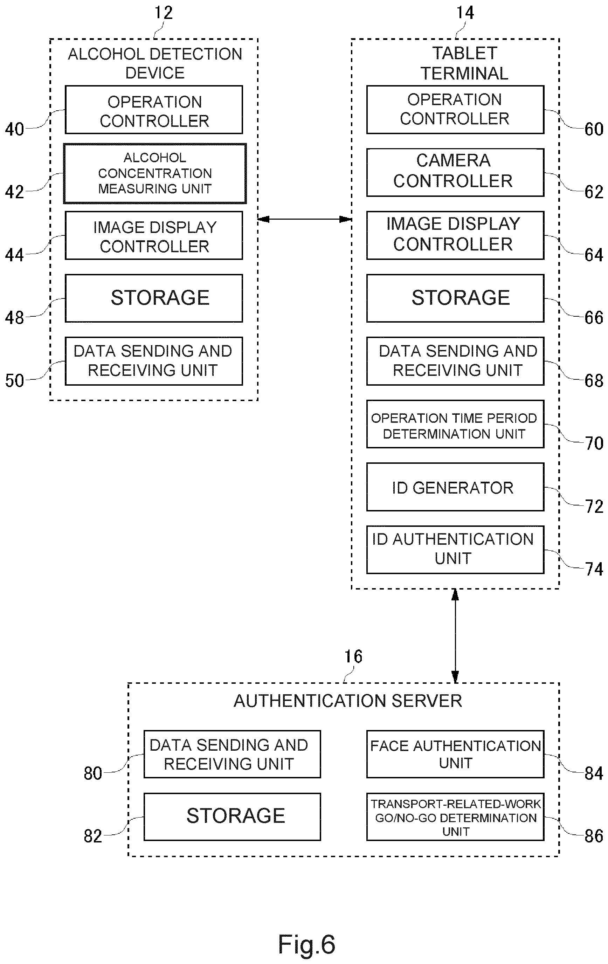

Figure 6 is a function block diagram of the alcohol detection system 10 of the embodiment. The same components in Figure 6 as in Figure 3 are designated by the same symbols, and their descriptions are omitted.

The tablet terminal 14 is provided with an ID generator 72 and an ID authentication unit 74.

The ID generator 72 generates an ID that the display 22 of the alcohol detection device 12 is caused to display, converts the generated ID (hereinafter referred to as the "generation ID") to a QR code, and sends it to the alcohol detection device 12 via the data sending and receiving unit 68.

A generation ID generated by the ID generator 72 is stored in the storage 66 for use in authentication. The ID generator 72 of the embodiment outputs, for example, randomly generated information as a generation ID. An example of the randomly generated information is a random number of a specified number of digits, and the random number may include numerics, characters, and symbols.

Using a QR code as an indication ID displayed on the display 22 of the tablet terminal 14 allows the indication ID to be given much more information as compared to a case in which a generation ID is displayed as it is in numeric value or the like. Note that using a QR code as an indication ID is just an example, and an indication ID may be displayed as a two-dimensional code other than a QR code or as a one-dimensional code such as a bar code. An indication ID may be a generation ID itself sent from the tablet terminal 14 without being converted to a one-dimensional or two-dimensional code.

The ID generator 72 of the embodiment generates a new ID each time the alcohol detection device 12 performs alcohol detection. In other words, an indication ID of the embodiment is a so-called one-time password. Then, based on a generation ID (a QR code) generated in the tablet terminal 14, the alcohol detection device 12 causes the display 22 to display an indication ID (the QR code). This prevents a person to be measured from getting to know the generation ID before alcohol detection, and therefore allows preventing frauds on the generation ID.

The ID generator 72 may not only generates an ID, but also, for example, choose one of a plurality of IDs stored in advance in the storage 48 of the alcohol detection device 12 each time alcohol detection is performed and send it to the alcohol detection device 12.

The ID authentication unit 74 extracts an indication ID from camera image information an image of which has been shot by the camera 32, and performs authentication based on the indication ID. Since in the embodiment an indication ID displayed on the alcohol detection device 12 is a QR code, the ID authentication unit 74 reads the indication ID by analyzing the QR code. The ID authentication unit 74 then determines whether the generation ID stored in the storage 66 and the extracted indication ID are identical or not, and determines the authentication to be successful if they are identical but be failed if they are not. In the following description, the determination by the ID authentication unit 74 is also called QR code authentication.

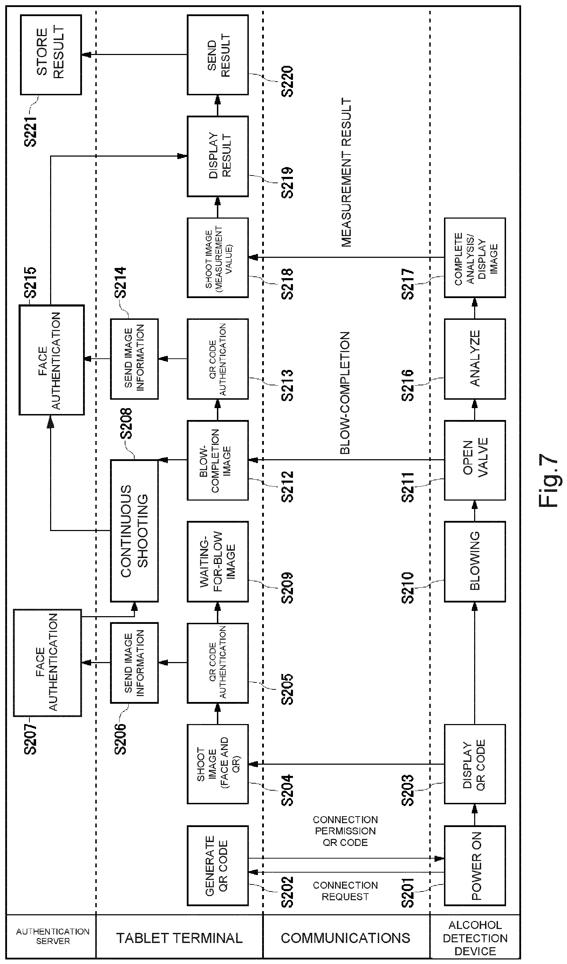

Figure 7 is a flowchart of an alcohol detection process of the embodiment. The alcohol detection process shown in Figure 7 is started by starting the alcohol detection app installed in the tablet terminal 14.

First, a person to be measured presses the power button of the alcohol detection device 12 to power on the alcohol detection device 12 (a step S201).

When powered on, the alcohol detection device 12 sends a connection request to the tablet terminal 14. When the tablet terminal 14 receives the connection request, the ID generator 72 generates a QR code (a step S202). The tablet terminal 14 then sends a connection permission along with the QR code to the alcohol detection device 12. That is, the QR code generated in the step S202 is a login ID. Note that the QR code may be generated not only when the tablet terminal 14 receives a connection request from the alcohol detection device 12, but also, for example, when the alcohol detection app is started in the tablet terminal 14.

Upon receiving the QR code, the alcohol detection device 12 displays the QR code as an indication ID on the display 22 (a step S203). In this way, the alcohol detection device 12 causes the display 22 to display an indication ID based on a QR code generated in the tablet terminal 14 each time alcohol detection is performed. This prevents the person to be measured from getting to know the indication ID before alcohol detection, and therefore allows preventing frauds on the indication ID.

Then, in the tablet terminal 14, the camera controller 62 controls the camera 32 so as to shoot an image of the face of the person to be measured and the QR code displayed on the display 22, and acquires camera image information (a step S204). The image shot by the camera 32 is displayed on the touch-panel display 30 of the tablet terminal 14 during the shooting, and the person to be measured adjusts the position of the person themself so that the face of the person themself and the QR code are included in the angle of view 36 of the camera 32.

Then, in the tablet terminal 14, the ID authentication unit 74 extracts the indication ID from the camera image information, and performs QR code authentication by comparing it with the generation ID stored in the storage 66 (a step S205). If the QR code authentication succeeds, the tablet terminal 14 sends the camera image information to the authentication server 16 (a step S206). If the QR code authentication fails, on the other hand, the tablet terminal 14 causes the touch-panel display 30 to display an image that indicates a failure of authentication.

The face authentication unit 84 of the authentication server 16 then performs face authentication based on the camera image information sent to the authentication server 16 (a step S207). The alcohol detection process proceeds to a step S208 if the face authentication succeeds. If the face authentication fails, on the other hand, the tablet terminal 14 causes the touch-panel display 30 to display an image that indicates a failure of authentication.

Note that it is not required to perform both the face authentication and the QR code authentication on a piece of camera image information in the step S204. For example, the camera 32 may acquire a plurality of camera image information in the step S204, one of the camera image information may be chosen and be subjected to only QR code authentication, and another of the camera image information may be chosen and be subjected to only face authentication. This is because there may be an image that is not appropriate for performing QR code authentication or face authentication depending on the state of image-shooting of camera image information (whether the focus is appropriate or not and the image-shooting area). That is, it is only required for the alcohol detection process of the embodiment to be able to perform face authentication and QR code authentication on a group of camera image information that are continuously shot images.

The success of face authentication then triggers the camera controller 62 to control the camera 32 so as to acquire camera image information that are continuously shot images of the face of the person to be measured (the step S208).

If the QR code authentication succeeds, the tablet terminal 14 causes the touch-panel display 30 to display the waiting-for-blow image (a step S209). When the person to be measured blows the breath (a step S210) and the breath is blown for a specified time, the solenoid valve of the alcohol detection device 12 is activated and the breath is sent into the alcohol concentration measuring unit 42 (a step S211). The alcohol detection device 12 sends the blow-completion signal indicating the completion of the blowing of the breath to the tablet terminal 14.

Upon receiving the blow-completion signal, the tablet terminal 14 causes the touch-panel display 30 to display the blow-completion image (a step S212). When the tablet terminal 14 receives the blow-completion signal, the camera controller 62 controls the camera 32 so as to finish the continuous shooting. A plurality of camera image information acquired by the continuous shooting are sent to the authentication server 16.

Upon receiving the blow-completion signal, the tablet terminal 14 also extracts a QR code from the camera image information acquired by the continuous shooting, and performs QR code authentication (a step S213). In the QR code authentication performed in the step S213, one or more camera image information are randomly chosen from the camera image information acquired by the continuous shooting and are subjected to QR code authentication.

As seen above, the alcohol detection process of the embodiment allows QR code authentication to be performed before alcohol detection is started and after it is finished, and therefore can more reliably prevent a person to be measured who performs alcohol detection from committing fraudulent acts. Note that QR code authentication may also be performed only once either before alcohol detection is started or after it is finished.

If the QR code authentication in the step S213 succeeds, the tablet terminal 14 sends the camera image information acquired by the continuous shooting to the authentication server 16 (a step S214).

When the authentication server 16 receives the camera image information acquired by the continuous shooting, the face authentication unit 84 performs face authentication based on the camera image information acquired by the continuous shooting (a step S215). The determination result of the face authentication is sent to the tablet terminal 14.

The activation of the solenoid valve in the step S209, on the other hand, causes the alcohol concentration measuring unit 42 to analyze the sent breath to calculate an alcohol measurement value (a step S216).

When the analysis of the breath alcohol concentration is completed, then the alcohol detection device 12 displays the alcohol measurement value, which is the analysis result, on the display 22, and sends the alcohol measurement value to the tablet terminal 14 (a step S217). Upon receiving the alcohol measurement value (the measurement result), the tablet terminal 14 shoots with the camera 32 an image of the alcohol measurement value displayed on the display 22 of the alcohol detection device 12 and the face of the person to be measured (a step S218).

Incidentally, for example, the alcohol detection process of the embodiment does not involve face authentication based on camera image information acquired by the image-shooting in the step S218 but makes the authentication server 16 store the camera image information as proof of an alcohol detection result. The embodiment, however, is not limited to this, and face authentication may be performed based on the camera image information acquired in the step S218.

The tablet terminal 14 also causes the touch-panel display 30 to display the face authentication result and the alcohol measurement value, causing the person to be measured to recognize them as the result of the alcohol detection (a step S219). In so doing, the tablet terminal 14 also displays determination results made by the operation time period determination unit 70 and the transport-related-work go/no-go determination unit 86 of the authentication server 16 on the touch-panel display 30. The tablet terminal 14 then sends the alcohol measurement value sent from the alcohol detection device 12, the camera image information including the alcohol measurement value acquired in the step S218, the camera image information acquired by the continuous shooting in the step S208, other determination results, and the like to the authentication server 16 (a step S220).

Upon receiving the various pieces of information sent by the tablet terminal 14 in the step S220, the authentication server 16 stores (saves) them in association with identification information that indicates the person to be measured (a step S221). This is the end of the alcohol detection process of the embodiment.

### (Third embodiment)

A third embodiment is described below. After alcohol detection is finished, the alcohol detection device 12 of the embodiment displays a QR code including information on the measurement result on the display 22, and the camera 32 acquires camera image information that is a shot image of the QR code displayed on the display 22. This allows the measurement result of the alcohol detection to be checked also through the camera image information acquired by the camera 32.

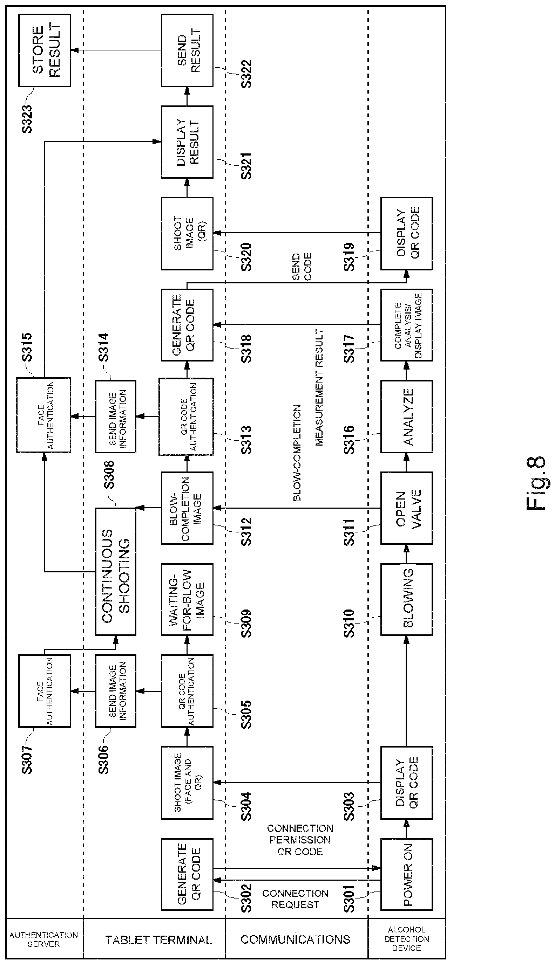

Figure 8 is a flowchart of an alcohol detection process of the embodiment. Steps S301 to S317 of the alcohol detection process shown in Figure 8 are the same as the steps S201 to S217 of the alcohol detection process shown in Figure 7, and therefore their descriptions are omitted. The same process as the alcohol detection process shown in Figure 7 is described with omissions.

When the alcohol detection device 12 completes the analysis of alcohol concentration in the step S317, an alcohol measurement value, which is the analysis result, is displayed on the display 22 of the alcohol detection device 12, and the alcohol measurement value (the measurement result) is sent to the tablet terminal 14.

Upon receiving the alcohol measurement value, the tablet terminal 14 newly generates a QR code that includes a login ID (a generation ID) generated in the step S302 and information on the measurement result (a step S318). The tablet terminal 14 then sends the generated QR code to the alcohol detection device 12.

In addition to the login ID and the measurement result, the QR code generated in the step S318 may include the name of a person to be measured who performed the alcohol detection, the date and time when the alcohol detection was performed, the manufacturing ID of the alcohol detection device 12 that performed the alcohol detection, determination results made by the operation time period determination unit 70 and the transport-related-work go/no-go determination unit 86, or other information.

Upon receiving the QR code, the alcohol detection device 12 displays the QR code on the display 22 (a step S319). The tablet terminal 14 then shoots with the camera 32 an image of the QR code displayed on the display 22 of the alcohol detection device 12 and the face of the person to be measured (a step S320).

Incidentally, for example, the alcohol detection process of the embodiment does not involve face authentication based on camera image information acquired by the image-shooting in the step S320 but makes the authentication server 16 store the camera image information as proof of an alcohol detection result. The embodiment, however, is not limited to this, and face authentication may be performed based on the camera image information acquired in the step S320.

The tablet terminal 14 also causes the touch-panel display 30 to display the face authentication result and the alcohol measurement value, causing the person to be measured to recognize them as the result of the alcohol detection (a step S321). In so doing, the tablet terminal 14 also displays determination results made by the operation time period determination unit 70 and the transport-related-work go/no-go determination unit 86 of the authentication server 16 on the touch-panel display 30. The tablet terminal 14 then sends the alcohol measurement value sent from the alcohol detection device 12, the camera image information including the QR code acquired in the step S320, the camera image information acquired by the continuous shooting in the step S308, other determination results, and the like to the authentication server 16 (a step S322).

Upon receiving the various information sent by the tablet terminal 14 in the step S322, the authentication server 16 stores (saves) them in association with identification information that indicates the person to be measured (a step S323). This is the end of the alcohol detection process of the embodiment.

While the disclosure has been described with reference to the above embodiments, the technical scope of the disclosure is not limited to the scope provided by the embodiments. Various modifications or improvements can be made to the embodiments without departing from the gist of the disclosure, and those added with the modifications or improvements are also included in the technical scope of the disclosure. The components and flowchart of each of the above embodiments may be embodied in combination with one another as appropriate.

For example, while a description has been made for the above embodiments on modes in which the breath alcohol concentration of a person to be measured is detected as biological information of the person to be measured, the embodiment is not limited to this, and other biological information may be used, for example, as long as the biological information can be detected from the breath of a person to be measured. Furthermore, the biological information is not limited to those that are detected from the breath of a person to be measured, and may be biological information that can be detected from a subject's sample such as blood, saliva, and part of a tissue.

While continuous shooting is the shooting of continuous still images in the above embodiments, the embodiment is not limited to this, and continuous shooting may be the shooting of a moving image. In this embodiment, an image at an arbitrary time is extracted from an acquired moving image to perform face authentication on a person to be measured and perform QR code authentication. The acquisition of camera image information with the camera 32 is not limited to the embodiment in which the camera image information is stored in the storage 66 of the tablet terminal 14 and is then sent to the authentication server 16. For example, an image shot by the camera 32 may be directly sent to the authentication server 16 without being stored in the tablet terminal 14, and be stored in the storage 82 of the authentication server 16.

While a description has been made for the above embodiments on modes in which the tablet terminal 14 generates a QR code and performs authentication on it, the embodiment is not limited to this. For example, a QR code may be generated by the alcohol detection device 12 or the authentication server 16, and may be authenticated by the authentication server 16.

While a description has been made for the above second and third embodiments on modes in which face authentication is performed after QR code authentication succeeds and the success of the face authentication triggers the camera 32 to perform continuous shooting, the embodiment is not limited to this. As an example of a variation, QR code authentication may be performed after face authentication succeeds and the success of the QR code authentication may trigger the camera 32 to perform continuous shooting.

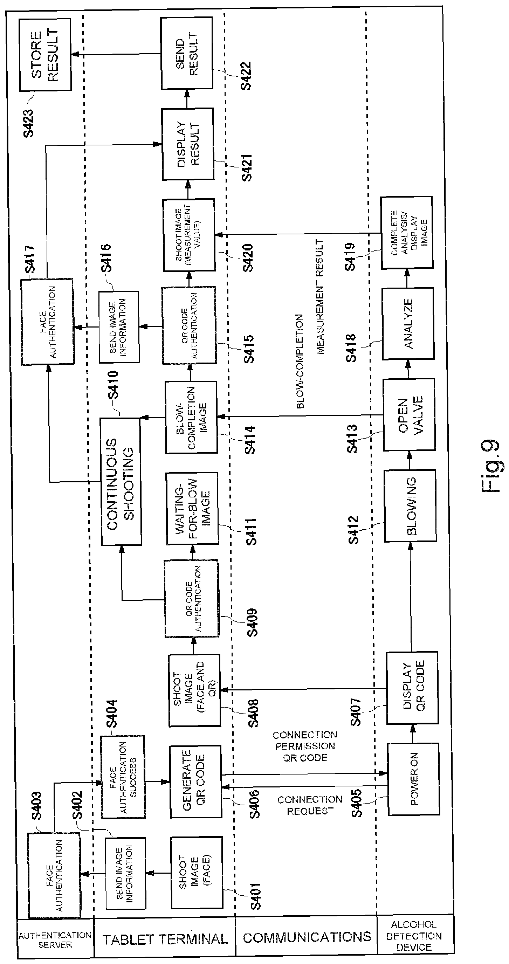

Figure 9 is a flowchart of an alcohol detection process of the variation. Steps S410 to S423 of the alcohol detection process shown in Figure 9 are the same as the steps S208 to S221 of the alcohol detection process shown in Figure 7, and therefore their descriptions are omitted. The same process as the alcohol detection process shown in Figure 7 is described with omissions.

First, the camera controller 62 of the tablet terminal 14 controls the camera 32 so as to shoot an image of the face of a person to be measured and acquires camera image information (a step S401), and the tablet terminal 14 sends the camera image information to the authentication server 16 (a step S402).

The face authentication unit 84 of the authentication server 16 then performs face authentication based on the camera image information sent to the authentication server 16 (a step S403). If the face authentication succeeds, the tablet terminal 14 receives a signal indicating the success of the face authentication (hereinafter referred to as a "face authentication success signal") from the authentication server 16 (a step S404).

The person to be measured then presses the power button of the alcohol detection device 12 to power on the alcohol detection device 12 (a step S405). The variation is not limited to this, and when the face authentication succeeds in the step S404, the tablet terminal 14 may send a start signal to the alcohol detection device 12, which may then starts upon receiving the start signal. Note that the alcohol detection device 12 before receiving a start signal is in so-called sleep in which the power is on but functions, for example, other than a function to receive signals are disabled.

When powered on, the alcohol detection device 12 sends a connection request to the tablet terminal 14. When the tablet terminal 14 receives the face authentication success signal from the authentication server 16 and the connection request from the alcohol detection device 12, the ID generator 72 generates a QR code (a step S406). The tablet terminal 14 then sends a connection permission along with the QR code to the alcohol detection device 12.

Upon receiving the QR code, the alcohol detection device 12 displays the QR code as an indication ID on the display 22 (a step S407). Then, in the tablet terminal 14, the camera controller 62 controls the camera 32 so as to shoot an image of the face of the person to be measured and the QR code displayed on the display 22, and acquires camera image information (a step S408).

Then, in the tablet terminal 14, the ID authentication unit 74 extracts the indication ID from the camera image information, and performs QR code authentication by comparing it with the generation ID stored in the storage 66 (a step S409). If the QR code authentication succeeds, the tablet terminal 14 starts the continuous shooting with the camera 32 in the step S410, and displays the waiting-for-blow image in the step S411.

The alcohol detection system 10 of the variation thus allows two types of authentication to be performed based on the indication ID and the face of the person to be measured, and therefore allows more reliably preventing the person to be measured who performs alcohol detection from committing fraudulent acts.

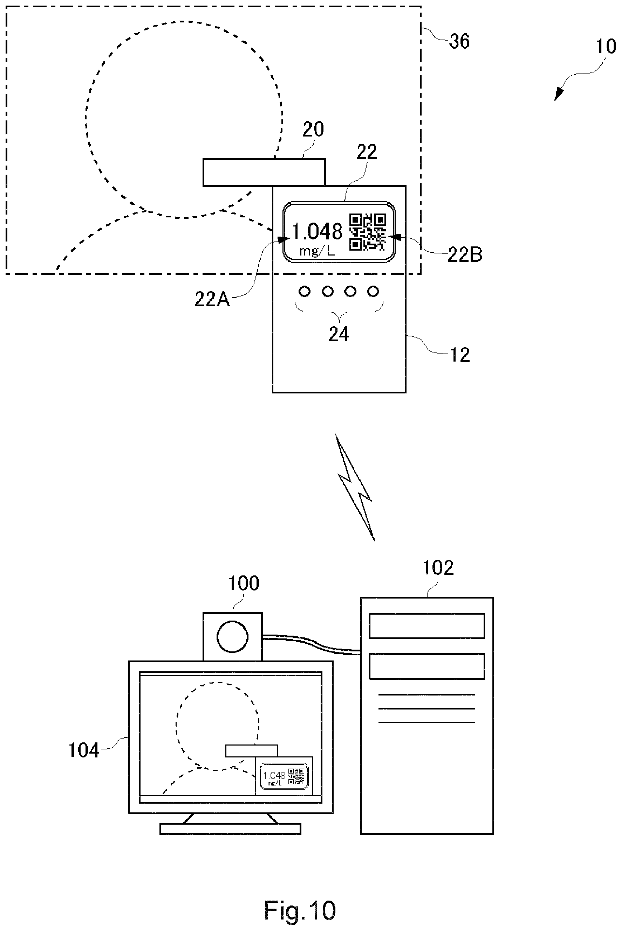

While a description has been made for the above embodiments on modes in which the alcohol detection system 10 comprises the alcohol detection device 12, the tablet terminal 14, and the authentication server 16, the embodiment is not limited to this. For example, the alcohol detection system 10 may comprise the alcohol detection device 12, a camera device 100, and an information-processing device 102 as shown in Figure 10.

The information-processing device 102 is provided with a display 104, and is connected with the camera device 100. That is, in the example in Figure 10, the information-processing device 102 is provided with the functions of the tablet terminal 14 and the authentication server 16 described in the above embodiments. In the example in Figure 10, the alcohol detection device 12 and the information-processing device 102 send and receive data between each other via wireless communication.

While a description has been made for the above embodiments on modes in which a person to be measured is a crew member of an aircraft, the embodiment is not limited to this. A person to be measured may be a driver of a business vehicle such as a bus, a taxi, a truck, and a motorcycle, or may be a crew member of a ship or a railroad train. A person to be measured is not limited to a crew member of an aircraft or a driver of a business vehicle, and may also be a worker working at or a manager managing a hospital, a factory, a store, an office, or the like.

Furthermore, while in the above embodiments the alcohol detection device 12 requires authentication using identification information (a QR code) and face authentication, the device that requires authentication is not limited to this and may be another device as long as it is a device to be used that requires authentication for use. That is, the authentication methods of the embodiments may be applied to an authentication system comprising a device to be used, an image-shooting device, and an information-processing device.

More specifically, the device to be used is provided with display means for displaying identification information, the camera shoots an image of the identification information displayed on the display means along with the face of a user of the device to be used, and the information-processing device performs authentication based on the identification information and the face of the user an image of which has been shot by the camera. Then, the success of the two types of authentication means that the user has succeeded in logging in to the device to be used, allowing the user to use the device to be used. This authentication system allows two types of authentication to be performed based on the identification information and the face of the person to be measured, and therefore allows preventing the user who uses the device to be used from committing fraudulent acts.

### DESCRIPTION OF THE SYMBOLS

10: Alcohol detection system (Authentication system)
12: Alcohol detection device (Biological information measuring device)
14: Tablet terminal (Information-processing device)
16: Authentication server (Information-processing device)
22: Display (Display means)
32: Camera (Image-shooting device)
72: ID authentication unit (Identification information authentication means)
86: Face authentication unit (Face authentication means)

## Claims

1. An authentication system comprising:
a biological information measuring device for measuring biological information of a person to be measured;
an image-shooting device; and
an information-processing device,
wherein the information-processing device is provided with face authentication means for performing face authentication on the person to be measured based on image information that indicates a face of the person to be measured an image of which has been shot by the image-shooting device, and
wherein the image-shooting device acquires image information that are continuously shot images of the face of the person to be measured on a condition that the face authentication succeeds before the biological information measuring device starts the measurement.

2. The authentication system according to claim 1,
wherein the face authentication means performs the face authentication before the biological information measuring device is powered on, and
wherein the biological information measuring device being powered on after the success of the face authentication triggers the image-shooting device to acquire image information that are continuously shot images of the face of the person to be measured.

3. The authentication system according to claim 1 or 2, wherein the image-shooting device finishes the continuous image-shooting after the biological information measuring device finishes the measurement, when acquisition of an object to be measured, which is a subject of the measurement, is started, or when the acquisition of the object to be measured is finished.

4. The authentication system according to any one of claims 1 to 3, wherein the image-shooting device acquires image information that is a shot image of a measurement value of the biological information displayed on the biological information measuring device.

5. The authentication system according to any one of claims 1 to 4,
wherein the biological information measuring device is provided with display means for displaying identification information,
wherein the image-shooting device shoots an image of the identification information displayed on the display means along with the face of the person to be measured, and wherein the information-processing device uses identification information authentication means to perform authentication based on the identification information an image of which has been shot by the image-shooting device, and uses the face authentication means to perform authentication based on the face of the person to be measured an image of which has been shot by the image-shooting device.

6. The authentication system according to claim 5, wherein the image-shooting device acquires image information that are continuously shot images of the face of the person to be measured if the authentication based on the identification information and the authentication based on the face of the person to be measured succeed.

7. The authentication system according to claim 5 or 6,
wherein the information-processing device generates the identification information each time the biological information measuring device performs the measurement, and
wherein the biological information measuring device causes the display means to display the identification information based on the identification information generated by the information-processing device.

8. The authentication system according to any one of claims 5 to 7, wherein the authentication based on the identification information is performed at least one of: before the biological information measuring device starts the measurement; while the biological information measuring device is performing the measurement; and after the biological information measuring device finishes the measurement.

9. The authentication system according to any one of claims 5 to 8,
wherein the biological information measuring device displays the identification information including information on a measurement result on the display means after finishing the measurement, and
wherein the image-shooting device acquires image information that is a shot image of the identification information displayed on the display means.

10. The authentication system according to any one of claims 1 to 9, wherein the biological information of the person to be measured is breath alcohol concentration of the person to be measured.

11. The authentication system according to any one of claims 1 to 10, wherein the face authentication means performs the face authentication based on a plurality of image information.

12. The authentication system according to any one of claims 1 to 11, wherein the face authentication means performs face authentication on the person to be measured with the image information and registered image information that is registered in advance and indicates the face of the person to be measured.

13. The authentication system according to any one of claims 1 to 12, wherein the biological information of the person to be measured is measured during a predetermined time period.

14. The authentication system according to claim 13,
wherein the person to be measured is a crew member of an aircraft, and
wherein the predetermined time period is at least one of specified time periods before and after an operation time period of the aircraft that the crew member boards for service.

15. An authentication system comprising:
a device to be used that requires authentication for use;
an image-shooting device; and
an information-processing device,
wherein the device to be used is provided with display means for displaying identification information,
wherein the image-shooting device shoots an image of the identification information displayed on the display means along with a face of a user of the device to be used, and wherein the information-processing device performs authentication based on the identification information and the face of the user an image of which has been shot by the image-shooting device.

16. An authentication method of a system comprising: a biological information measuring device for measuring biological information of a person to be measured; an image-shooting device; and an information-processing device, the authentication method having:
a first step of the information-processing device performing face authentication on the person to be measured based on image information that indicates a face of the person to be measured an image of which has been shot by the image-shooting device; and
a second step of the image-shooting device acquiring image information that are continuously shot images of the face of the person to be measured on a condition that the face authentication succeeds before the biological information measuring device starts the measurement.

17. An authentication method of a system comprising: a biological information measuring device for measuring biological information of a person to be measured; an image-shooting device; and an information-processing device, the authentication method having:
a first step of the biological information measuring device displaying identification information on display means;
a second step of the image-shooting device shooting an image of the identification information displayed on the display means along with a face of the person to be measured, and
a third step of the information-processing device using identification information authentication means to perform authentication based on the identification information an image of which has been shot by the image-shooting device, and using face authentication means to perform authentication based on the face of the person to be measured an image of which has been shot by the image-shooting device.

18. An authentication program of a system comprising: a biological information measuring device for measuring biological information of a person to be measured; an image-shooting device; and an information-processing device, the authentication program causing a computer to execute:
a first step of the information-processing device performing face authentication on the person to be measured based on image information that indicates a face of the person to be measured an image of which has been shot by the image-shooting device; and
a second step of the image-shooting device acquiring image information that are continuously shot images of the face of the person to be measured on a condition that the face authentication succeeds before the biological information measuring device starts the measurement.

19. An authentication program of a system comprising: a biological information measuring device for measuring biological information of a person to be measured; an image-shooting device; and an information-processing device, the authentication program causing a computer to execute:
a first step of the biological information measuring device displaying identification information on display means;
a second step of the image-shooting device shooting an image of the identification information displayed on the display means along with a face of the person to be measured, and
a third step of the information-processing device using identification information authentication means to perform authentication based on the identification information an image of which has been shot by the image-shooting device, and using face authentication means to perform authentication based on the face of the person to be measured an image of which has been shot by the image-shooting device.

20. A computer-readable non-transitory storage medium holding the authentication program according to claim 18.

21. A computer-readable non-transitory storage medium holding the authentication program according to claim 19.
